# EUROPEAN PATENT SPECIFICATION

(11) **EP 0 749 490 B1**
(45) Date of publication and mention of the grant of the patent: **27.12.2006**
(21) Application number: 95913607.8
(22) Date of filing: 10.03.1995
(51) Int. Cl.: C12N 15/82, C12N 15/32, C07K 14/435, A01N 63/02, A01H 5/00

(54) **EXPRESSION OF $i(BACILLUS THURINGIENSIS CRY) PROTEINS IN PLANT PLASTIDS**
EXPRESSION DER BAZILLUS THURINGIENSIS CRY-PROTEINE IN PFLANZEN-PLASTIDEN
EXPRESSION DE PROTEINES $i(CRY) DU $i(BACILLUS THURIGIENSIS) DANS DES PLASTES DE PLANTES

(30) Priority: 11.03.1994 US 209951
(43) Date of publication of application: 27.12.1996
(73) Proprietor: Calgene LLC, Davis, CA 95616 (US); STATE UNIVERSITY OF NEW JERSEY RUTGERS, Piscataway, NJ 08855-1179 (US)
(72) Inventor: McBRIDE, Kevin E., Davis, CA 95616 (US); MALIGA, Pal, East Brunswick, NJ 08816 (US)
(74) Representative: Helbing, Jörg
(86) International application number: PCT/US1995/002900
(87) International publication number: WO 1995/024492

(56) References cited:
- EP-A- 0 142 924
- EP-A- 0 193 259
- WO-A-90/10076
- WO-A-95/24493
- CURR MICROBIOL 21 (5). 1990. 283-288., CHUNGJATUPORNCHAI W 'EXPRESSION OF THE MOSQUITOCIDAL-PROTEIN GENES OF BACILLUS THURINGIENSIS-SSP-ISRAELENSIS AND THE HERBICIDE-RESISTANCE GENE BAR IN SYNECHOCYSTIS PCC6803'
- PLANT MOL BIOL 20 (1). 1992. 81-93., WONG E Y, ET AL. 'ARABIDOPSIS-THALIANA SMALL SUBUNIT LEADER AND TRANSIT PEPTIDE ENHANCES THE EXPRESSION OF BACILLUS-THURINGIENSIS PROTEINS IN TRANSGENIC PLANTS.'
- THE EMBO JOURNAL, vol. 4, no. 6, 1985 pages 1367-1372, DE BLOCK, M., ET AL. 'CHLOROPLAST TRANSFORMATION BY AGROBACTERIUM TRANSFORMATION'
- PLANT GENE RESEARCH: BASIC KNOWLEDGE AND APPLICATION , PLANT DNA INFECTIOUS AGENTS, SPRINGER VERLAG, 1987. ED. T HOHN, ET AL. CHAPTER 14, pages 311-320, CORNELISSEN, M.J., ET AL. 'PLASTID TRANSFORMATION: A PROGRESS REPORT'
- BIOTECHNOLOGY, vol. 9, no. 11, November 1991 page 1103 VENKATESWARLU, K., ET AL. 'EVIDENCE FOR T-DNA MEDIATED GENE TARGETING TO TOBACCO CHLOROPLASTS'
- PROCEEDINGS OF THE NATIONAL ACADEMY OF SCIENCES OF USA, vol. 88, April 1991 WASHINGTON US, pages 3324-3328, PERLAK, F.J., ET AL. 'MODIFICATION OF THE CODING SEQUENCES ENHANCES PLANT EXPRESSION OF INSECT CONTROL PROTEIN GENES'
- BIOTECHNOLOGY, vol. 8, no. 10, October 1990 PERLAK, F.J., ET AL. 'INSECT RESISTANT COTTON PLANTS'
- TRENDS IN BIOTECHNOLOGY, vol. 11, March 1993 pages 101-107, MALIGA, P. 'TOWARDS PLASTID TRANSFORMATION IN FLOWERING PLANTS'
- PROCEEDINGS OF THE NATIONAL ACADEMY OF SCIENCES OF USA, vol. 90, 1993 WASHINGTON US, pages 913-917, SVAB, Z., ET AL. 'HIGH-FREQUENCY PLASTID TRANSFORMATION IN TOBACCO BY SELECTION FOR A CHIMERIC AADA GENE'
- THE EMBO JOURNAL, vol. 12, no. 2, 1993 pages 601-606, STAUB, J.M., ET AL. 'ACCUMULATION OF D1 POLYPEPTIDE IN TOBACCO PLASTIDS IS REGULATED VIA THE UNTRANSLATED REGION OF THE PSBA MRNA'
- MOLECULAR AND GENERAL GENETICS, vol. 241, 1993 BERLIN DE, pages 49-56, CARRER, H., ET AL. 'KANAMYCIN RESISTANCE AS A SELECTABLE MARKER FOR PLASTID TRANSFORMATION IN TOBACCO'

## Description

### Field of the Invention

This invention relates to the application of genetic engineering techniques to plants. More specifically, the invention relates to compositions and methods expressing insecticidal *Bacillus thuringiensis* toxin proteins in plant plastids.

### Background

Plastids of higher plants, *i.e,* chloroplasts, amyloplasts and chromoplasts, have the same genetic content, and thus are believed to be derived from a common precursor, known as a proplastid. The plastid genome is circular and varies in size among plant species from about 120 to about 217 kilobase pairs (kb). The genome typically includes a large inverted repeat, which can contain up to about 76 kilobase pairs, but which is more typically in the range of about 20 to about 30 kilobase pairs. The inverted repeat present in the plastid genome of various organisms has been described (Palmer, J. D. (1990) Trends Genet. 6:115-120).

One advantage of plant plastid transformation over nuclear transformation is that the plastids of most plants are maternally inherited, and consequently heterologous plastid genes are not pollen disseminated. This feature is particularly attractive for transgenic plants having altered agronomic traits, as introduced resistance or tolerance to natural or chemical conditions will not be transmitted to wild-type relatives.

Plant plastids are also major biosynthetic centers. In addition to photosynthesis in chloroplasts, plastids are responsible for production of important compounds such as amino acids, complex carbohydrates, fatty acids, and pigments.

Plastids can be present in a plant cell at a very high copy number, with up to 50,000 copies per cell present for the chloroplast genome (Bendich, A. J. (1987) *BioEssays* 6:279-282). Thus, through plastid transformation plant cells can be engineered to maintain an introduced gene of interest at a very high copy number.

For all of the above reasons, the plastids of higher plants present an attractive target for genetic engineering. Stable transformation of plastids has been reported in the green algae *Chlamydomonas* (Boynton *et al.* (1988) *Science 240*:1534-1538) and more recently in higher plants (Svab et *al.* (1990) Proc. *Natl. Acad. Sci. USA* 87:8526-8530; Svab and Maliga (1993) *Proc. Natl. Acad. Sci. USA 90*:913-917 ; (Staub, J. M. and Maliga, P. (1993), *EMBO J*. *12*:601-606). The method disclosed for plastid transformation in higher plants relies on particle gun delivery of DNA containing a selectable marker and targeting of the DNA to the plastid genome through homologous recombination.

There is a continuing need to introduce newly discovered or alternative *Bacillus thuringiensis* genes into crop plants. Cry proteins (d-endotoxins) from *Bacillus thuringiensis* have potent insecticidal activity against a number of Lepidopteran, Dipteran, and Coleopteran insects. These proteins are classified CryI to CryV, based on amino acid sequence homology and insecticidal activity. Most CryI proteins are synthesized as protoxins (ca. 130-140 kDa) then solubilized and proteolytically processed into active toxin fragments (ca. 60-70 kDa).

The poor expression of the protoxin genes from the nucleus of plants has heretofore required the use of 'truncated' versions of these genes. The truncated versions code only for the active toxin fragments. Other attempts to increase the expression efficiency have included resynthesizing the *Bacillus thuringiensis* toxin genes to utilize plant preferred codons. Many problems can arise in such extensive reconstruction of these large *cry* genes (approximately 3.5 Kb), and the process is both laborious and expensive.

The expression of the modified gene for a truncated form of the crylA(c) gene, encoding the insecticidal portion of the lepidopteran-active CrylA(c) protein from Bacillus thuringiensis var. kurstaki (B.t.k.) HD73, under control of the Arabidopsis thaliana ribulose-1,5-bisphosphate carboxylase (Rubisco) small subunit atsIA promoter with and without its associated transit peptide was analyzed in transgenic tobacco plants (Wong, E.Y., et al. Plant Molecular Biology 20:81-93, 1992).

Problems can also arise as new insect pests become endemic, or as existing populations develop resistance to a particular level or type of *Bacillus thuringiensis* toxin. Thus, there is a particular need for producing higher and thereby more effective levels of the *Bacillus thuringiensis* toxin in plants, a need which will only increase with time.

### SUMMARY OF THE INVENTION

By this invention, plastid expression constructs are provided which are useful for genetic engineering of plant cells and which provide for enhanced expression of the *Bacillus thuringiensis* cry proteins in plant cell plastids. The transformed plastids should be metabolically active plastids, and are preferably maintained at a high copy number in the plant tissue of interest, most preferably the chloroplasts found in green plant tissues, such as leaves or cotyledons.

The plastid expression constructs for use in this invention generally include a plastid promoter region, a DNA sequence encoding a *Bacillus thuringiensis* cry protein, and a transcription termination region capable of terminating transcription in a plant plastid.

The plastid expression construct of this invention is furthermore linked to a construct having a DNA sequence encoding a selectable marker which can be expressed in a plant plastid. Expression of the selectable marker allows the identification of plant cells comprising a plastid expressing the marker.

Moreover, for transfer of the construct into a plant cell the construct includes means for inserting the expression and selection constructs into the plastid genome., namely DNA regions of homology to the target plastid genome which flank the constructs.

The construct of the invention comprises a native *Bacillus thuringiensis* DNA encoding sequence, which may be a sequence encoding the protoxin. In a preferred embodiment, the DNA encoding sequence is the *cry*IA(c) gene.

Plant cell plastids containing the construct are also contemplated in the invention, as are plants, plant seeds, plant cells or progeny thereof containing plastids comprising the conStruct. A preferred plant for this purpose is tobacco.

The invention also includes a method for the expression of an insecticidal *Bacillus thuringiensis* toxin in a plant cell, by expressing the *Bacillus thuringiensis* toxin in plastids of the plant cell from the construct of present invention. By this method the expression is enhanced separate from that enhancement which occurs from the increased copy number in plastid transformation.

By this invention the insecticidal *Bacillus thuringiensis* toxin is produced in plastids of a plant cell from the native DNA encoding sequence, with enhanced levels of expression of an insect resistant phenotype, as measured by insect feeding assays.

### DESCRIPTION OF THE FIGURES

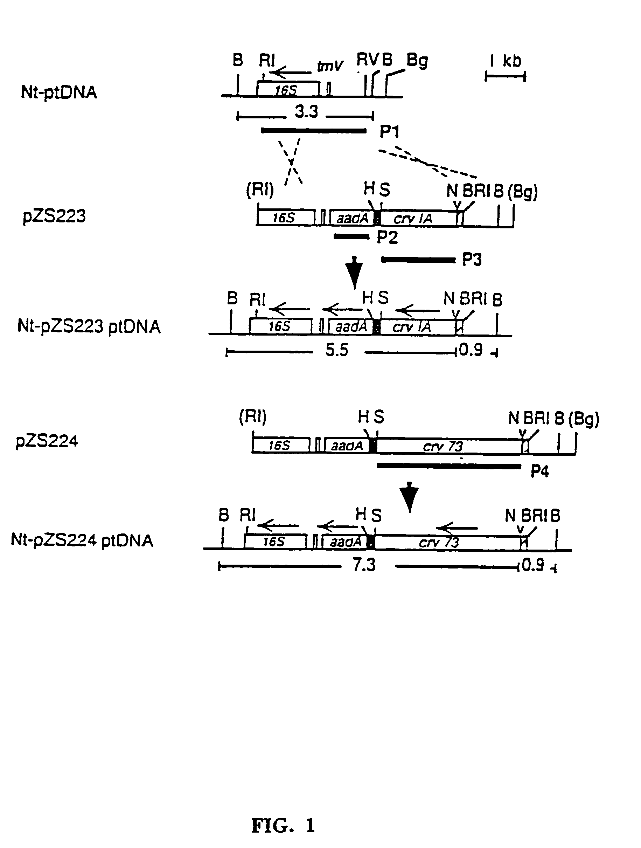

Figure 1 shows integration of cry genes from vectors pZS223 and pZS224 into the wild-type plastid genome (Nt-ptDNA) to yield transplastomes Nt-pZS223 ptDNA and Nt-pZS224 ptDNA, respectively.

### DETAILED DESCRIPTION OF THE INVENTION

A plastid expression construct of this invention generally comprises a promoter functional in a plant plastid, a DNA sequence encoding a *Bacillus thuringiensis* cry protein, and a transcription termination region capable of terminating transcription in a plant plastid. These elements are provided as operably joined components in the 5' to 3' direction of transcription.

In developing the constructs the various fragments comprising the regulatory regions and open reading frame may be subjected to different processing conditions, such as ligation, restriction enzyme digestion, PCR, in vitro mutagenesis, linkers and adapters addition.
Thus, nucleotide transitions, transversions, insertions or deletions may be performed on the DNA which is employed in the regulatory regions or the DNA sequences of interest for expression in the plastids. Methods for restriction digests, Klenow blunt end treatments, and ligations are well known to those in the art and are described, for example, by Maniatis *et al.* (in *Molecular cloning: a laboratory manual* (1982) Cold Spring Harbor Laboratory Press, Cold Spring Harbor, NY).

During the preparation of the constructs, the various fragments of DNA will often be cloned in an appropriate cloning vector, which allows for amplification of the DNA, modification of the DNA or manipulation of the DNA by joining or removing sequences or linkers. Preferably, the vectors will be capable of replication to , at least a relatively high copy number in *E*. *coli.* A number of vectors are readily available for cloning, including such vectors as pBR322, vectors of the pUC series, the M13 series vectors, and pBluescript vectors (Stratagene; La Jolla, CA).

In order to provide a means of selecting the desired plant cells, vectors for plastid transformation contain a construct which provides for expression of a selectable marker gene. Marker genes are plant-expressible DNA sequences which express a polypeptide which resists a natural inhibition by, attenuates, or inactivates a selective substance, *i.e.*, antibiotic or herbicide.

Alternatively, a marker gene may provide some other visibly reactive response, *i.e.,* may cause a distinctive appearance or growth pattern relative to plants or plant cells not expressing the selectable marker gene in the presence of some substance, either as applied directly to the plant or plant cells or as present in the plant or plant cell growth media.

In either case, the plants or plant cells containing such selectable marker genes will have a distinctive phenotype for purposes of identification, i.e., they will be distinguishable from non-transformed cells. The characteristic phenotype allows the identification of cells, cell groups, tissues, organs, plant parts or whole plants containing the construct.

Detection of the marker phenotype makes possible the selection of cells having a second gene to which the marker gene has been linked. This second gene typically comprises a desirable phenotype which is not readily identifiable in transformed cells, but which is present when the plant cell or derivative thereof is grown to maturity, even under conditions wherein the selectable marker phenotype itself is not apparent.

The use of such a marker for identification of plant cells containing a plastid construct has been described. Svab *et al.* (1993 supra). In the examples provided below, a bacterial *aad*A gene is expressed as the marker under the regulatory control of chloroplast 5' promoter and 3' transcription termination regions, specifically the tobacco 16S rRNA promoter *rrn* region and *rps*16 3' termination region. Numerous additional promoter regions may also be used to drive expression of the selectable marker gene, including various plastid promoters and bacterial promoters which have been shown to function in plant plastids.

Expression of the *aad*A gene confers resistance to spectinomycin and streptomycin, and thus allows for the identification of plant cells expressing this marker. The *aad*A gene product allows for continued growth and greening of cells whose chloroplasts comprise the selectable marker gene product. Cells which do not contain the selectable marker gene product are bleached. Selection for the *aad*A gene marker is thus based on identification of plant cells which are not bleached by the presence of streptomycin, or more preferably spectinomycin, in the plant growth medium.

A number of markers have been developed for use with plant cells, such as resistance to chloramphenicol, the aminoglycoside G418 or hygromycin. Other genes which encode a product involved in chloroplast metabolism may also be used as selectable markers. For example, genes which provide resistance to plant herbicides such as glyphosate, bromoxynil or imidazolinone may find particular use. Such genes have been reported (Stalker *et al., J. Biol. Chem.* (1985) *260*:4724-4728 (glyphosate resistant EPSP); Stalker *et al., J. Biol. Chem.* (1985) *263*:6310-6314 (bromoxynil resistant nitrilase gene); and Sathasivan et *al., Nucl. Acids Res.* (1990) *18*:2188 (AHAS imidazolinone resistance gene)).

Stable transformation of tobacco plastid genomes by particle bombardment is reported (Svab *et*.*al*. (1990 *supra*) and Svab *et al.* (1993 *supra*)). The methods described therein may be employed to obtain plants homoplasmic for plastid expression constructs.

Generally, bombarded tissue is cultured for approximately 2 days on a cell division-promoting media, after which the plant tissue is transferred to a selective media containing an inhibitory amount of the particular selective agent, as well as the particular hormones and other substances necessary to obtain regeneration for that particular plant species. Shoots are then subcultured on the same selective media to ensure production and selection of homoplasmic shoots.

Homoplasmy is verified by southern analysis. In the examples provided below, *Bam*HI-digested total cellular DNA is tested with various probes, specifically, a part of the plastid targeting fragment, an *aad*A fragment, a 1.8 kb *cry*1A fragment and a 3.5 kb fragment of the *cry*73 coding region. Southern blot analysis with these probes confirms the integration of the chimeric cry genes in the tobacco plastid genome to yield transplastome lines.

As an alternative to a second round of shoot formation, the initial selected shoots may be grown to mature plants and segregation relied upon to provide transformed plants homoplastic for the inserted gene construct.

Where transformation and regeneration methods have been adapted for a given plant species, either by *Agrobacterium*-mediated transformation, bombardment or some other method, the established techniques may be modified for use in selection and regeneration methods to produce plastid-transformed plants. For example, the methods described herein for tobacco are readily adaptable to other solanaceous species, such as tomato, petunia and potato.

In *Brassica, Agrobacterium*-mediated transformation and regeneration protocols generally involve the use of hypocotyl tissue, a non-green tissue which might contain a low plastid content. Thus, for *Brassica,* preferred target tissues would include microspore-derived hypocotyl or cotyledonary tissues (which are green and thus contain numerous plastids) or leaf tissue explants. While the regeneration rates from such tissues may be low, positional effects, such as seen with *Agrobacterium*-mediated transformation, are not expected, thus it would not be necessary to screen numerous successfully transformed plants in order to obtain a desired phenotype.

For cotton, transformation of *Gossypium hirsutum* L. cotyledons by co-cultivation with *Agrobacterium tumefaciens* has been described by Firoozabady *et al., Plant Mol. Bio.* (1987) *10*:105-116 and Umbeck *et al., Bio*/*Technology* (1987) *5*:263-266. Again, as for *Brassica,* this tissue may contain insufficient plastid content for chloroplast transformation. Thus, as for *Brassica,* an alternative method for transformation and regeneration of alternative target tissue containing chloroplasts may be desirable, for instance targeting green embryogenic tissue.

Other plant species may be similarly transformed using related techniques. Alternatively, microprojectile bombardment methods, such as described by Klein *et al.* (*Bio*/*Technology 10*:286-291) may also be used to obtain nuclear transformed plants comprising the viral single subunit RNA polymerase expression constructs described herein. Cotton transformation by particle bombardment is reported in WO 92/15675, published September 17, 1992.

The vectors for use in plastid transformation include means for providing a stable transfer of the plastid expression construct and selectable marker construct into the plastid genome. This is provided by regions of homology to the target plastid genome. The regions of homology flank the construct to be transferred and provide for transfer to the plastid genome by homologous recombination, via a double crossover into the genome. The complete DNA sequence of the plastid genome of tobacco has been reported (Shinozaki *et al.* (1986) *EMBO J.* 5:2043-2049). Complete DNA sequences of the plastid genomes from liverwort (Ohyama *et al.* (1986) *Nature 322*:572-574) and rice (Hiratsuka *et al.* (1989) *Mol. Gen. Genet*. *217*:185-194), have also been reported.

Where the regions of homology are present in the inverted repeat regions of the plastid genome (known as IRA and IRB), two copies of the transgene are expected per transformed plastid. The regions of homology within the plastid genome are approximately 1kb in size. Smaller regions of homology may also be used, and as little as 100 bp can provide for homologous recombination into the plastid genome. However, the frequency of recombination and thus the frequency of obtaining plants having transformed plastids decreases with decreasing size of the homology regions.

Examples of constructs having regions of homology the plastid genome are described in Svab *et.al*. (1990 supra) and Svab *et al*. (1993 supra). Regions useful for recombination into tobacco and *Brassica* plastid genomes are also identified in the following examples, but homologous recombination and selection constructs may be prepared using many plastid DNA sequences, and to any target plant species. In the examples provided herein, the flanking tobacco plastid homology regions of the plastid expression construct direct the insertion of a *Bacillus thuringiensis* transgene into the tobacco genome between *trn*V and the *rps*12 operon. Since integration into the plastid genome occurs by homologous recombination and the target site is in an inverted repeat region of the plastid genome, two copies of the transgene per plastid genome are expected. Selection is made for the spectinomycin resistance marker phenotype expressed by the *aad*A gene.

In the example the native cry gene, *i.e*., having an unmodified coding region to the protoxin, is placed into a plastid expression construct for expression of *Bacillus thuringiensis* toxin from the plant plastid.

A synthetic *Bacillus thuringiensis* gene is placed in the same expression construct as the protoxin gene. The synthetic gene is designed to have tobacco RuBPCO small subunit codon usage, with an overall increase in the guanine plus cytosine content from 39% to 55% with respect to the native gene, and has been truncated to leave only those sequences which enode the active fragment of the toxin. Such a gene is known to provide optimal expression from the plant nuclear genome.

Both encoding sequences are introduced via a chloroplast transformation vector (Fig. 1). Tobacco lines containing the native encoding sequence to the protoxin demonstrate strong insecticidal bioactivity, as measured by insect feeding assays. In transformed plants containing the native encoding sequence, the *Bacillus thuringiensis* toxin is present as a component of up to about 5% or greater of the total leaf protein, a level which is much higher than is present in the leaf of plants resulting from nuclear transformation.

Tobacco lines having a synthetic *cry*IA(c) gene demonstrate no observable bioactivity. In plants containing the gene resynthesized to approximate the preferred codons of the plant genome, the mRNA to the toxin appears degraded, and little or no toxin protein appears present in the leaf.

It is now shown that the native *Bacillus thuringiensis* gene achieves an expression level which is much higher in plastid expression than is possible with resynthesized sequence to the same gene, thus demonstrating that a gene having native bacterial encoding sequence can achieve high levels of expression in a plant plastid. The above results eliminate the need to resynthesize the *Bacillus thuringiensis* toxin genes to achieve high level expression in plants.

### EXAMPLES

In the experimental disclosure which follows, all temperatures are given in degrees centigrade (°), weights are given in grams (g), milligram (mg) or micrograms (µg), concentrations are given as molar (M), millimolar (mM) or micromolar (µM) and all volumes are given in liters (1), milliliters (ml) or microliters (µl), unless otherwise indicated.

### EXAMPLE 1. PLASTID TRANSFORMATION VECTORS

Constructs and methods for use in transforming the plastids of higher plants are described in Svab *et al.* (1990 *supra*), Svab et *al.* (1993 supra) and Staub *et al.* (1993 *supra*). The complete DNA sequences of the plastid genome of tobacco are reported by Shinozaki *et al.* (1986 *supra*). All plastid DNA references in the following description are to the nucleotide number from tobacco.

The *cry*IA(c) gene is obtained from plasmid pBtkHD73 (Toagosei Chemical Co., Japan). This gene is further processed by digestion with *Sma*I/*Nsi*I and a synthetic adapter is inserted (top strand: 5'-CCCGGATCCATGGATAACAATCCGA-ACATCAATGAATGCA-3'; bottom strand: 5'-TTCATTGATGTTCGGATT-GTTATCCATGGATCCGGG-3'). The entire 5' untranslated region from the *cry*IA(c) gene is then removed, and an *Nco*I site is introduced at the natural start codon (position 163 of the nucleotide sequence (Adang et *al.* (1985) *Gene* 36;289-300). A *Bam*HI site is introduced just upstream of the NcoI site. Oligonucleotide mutagenesis is performed to introduce *Bgl*II and *Sal*I sites directly adjacent to the stop codon of the *cry*IA(c) gene, to facilitate removal of unwanted DNA 3' of the coding region. The remaining sequence includes the entire encoding region to the protoxin.

A synthetic *cry*IA(c) gene encoding the active toxin fragment is constructed by annealing and ligating 70 and 90 base oligonucleotides, in a method as described (Wosnick *et al.* (1987) Gene *60*;115-127). The synthetic gene is designed to have tobacco RuBISCO small subunit codon usage, including a guanine and cytosine content of 55%, with an *NcoI* site at the start codon and *a Sal*I site at the stop codon, while still encoding the amino acid sequence of the toxin. This synthetic gene is also truncated, however, so that the encoding region only provides the amino acid sequence to the active fragment of the protoxin.

A plastid transformation vector is used which carries a passenger gene in a *Prrn*(L)*rbcL*(*S*)/*Trps*16 expression cassette, with polylinker restriction sites. The *Prrn*(L)*rbcL*(S) fragments are described in Svab *et al.* (1993 *supra*)*.* To further secure the stability of the mRNAs, the *Trps*16 fragment is cloned downstream of the passenger gene encoding region. The *Trps*16 fragment comprises the *rps*16 gene 3'-regulatory region from nucleotides 5,087 to 4,939 in the tobacco plasmid DNA.

Chimeric genes are preferably inserted into the vector to direct their transcription towards the rrn operon. Thus, in the plastid genome, chimeric genes are transcribed from the Prrn(L)rbcL(S) 5'-regulatory region comprising the long rrn operon promoter fragment from nucleotides 102,561 to 102,677 of the tobacco plastid genome, which is fused with a synthetic leader sequence designed after the *rbc*L gene leader between nucleotides 57,569 to 57,584 in the plastid DNA.

The plastid transformation vector also carries a selectable spectinomycin resistance gene (*aad*A) under control of *psb*A gene expression signals. The regulatory and encoding sequences are also flanked by plastid DNA homology regions whose limits are bp 138,447 (EcoRI) to 140,219 (*Hinc*II) and 140,219 (*Hinc*II) to 141,382 (*Bgl*II) of the tobacco plastid genome (Shinozaki *et al.* (1986 *supra)).* This directs insertion of foreign genes located between the flanking regions into the plastid between the *trn*V gene and the *rps*12/7 operon.

This plastid transformation vector is digested with the *Nco*I/*Sal*I restriction endonucleases to remove the encoding region of the passenger gene, which is then replaced with a *Nco*I/*Sal*I fragment containing the synthetic *cry*IA(c) coding region, yielding a vector which is designated pZS223 (Fig. 1). The wild type *cry*IA(c) protoxin gene is similarly cloned as an N*co*I/*Sal*I fragment, yielding a plasmid designated pZS224. By this approach *Bacillus thuringiensis* DNA 3' of the protein coding region is omitted for both plasmids, pZS223 and pZS224.

The insertion of the respective cry genes from vectors pZS223 and pZS224 into the wild-type plastid genome (Nt-ptDNA) to yield transplastomes Nt-pZS223 and Nt-pZS224, respectively, is shown in Fig. 1. The abbreviations used in Fig. 1 are as follows: 16S, 16S rRNA gene; *trnV, trnV* gene; *aad*A, spectinomycin resistance gene; *cry*1A and *cry*73 are synthetic and native *Bacillus thuringiensis* d-endotoxin genes, respectively. The restriction endonuclease cleavage sites are designated as follows: B, *Bam*HI; Bg, *Bgl*II; H, HindIII; N, *Nco*I; RI, *Eco*RI, RV, *Eco*RV; S, *Sal*I.

### EXAMPLE 2. PLANT PLASTID TRANSFORMATION

Stable transformation of tobacco plastid genomes by particle bombardment is reported in Svab et.al. (1990 *supra)* and Svab *et al.* (1993 *supra).* The methods described therein may be employed to obtain plants transformed with the plastid expression constructs described herein. Such methods generally involve DNA bombardment of a target host explant, preferably an explant made from a tissue which is rich in metabolically active plastids, such as green plant tissues including leaves or cotyledons.

Tobacco seeds (N. tabacum v. Xanthi N/C) are surface sterilized in a 50% chlorox solution (2.5% sodium hypochlorite) for 20 minutes and rinsed 4 times in sterile H₂O. These are plated asceptically on a 0.2x MS salts media and allowed to germinate. The seedlings are grown on agar solidified MS media with 30g/l sucrose (Murashige *et al.* (1962) *Physiol. Plant 15*:493-497).

Tungsten microprojectiles (1.0µM) are coated with plasmid DNA according to Maliga (Maliga, P. (1993) Methods in Plant Molecular Biology - A Laboratory Manual, eds. Pal Maliga, Daniel Klessig, Anthony Cashmore, Wilhelm Gruissem and Joseph Varner; Cold Spring Harbor Press) and used to bombard mature leaves, placed abaxial side up on RMOP media; MS salts, 1 mg/l BAP, 0.1 mg/l NAA, 30 g/l sucrose and 0.7% phytagar. Svab *et al.* (1990) *Proc. Natl. Acad. Sci. USA 87*:8526-8530 (using the Bio-Rad PDS 1000 He system (Sanford *et al.,* An improved, helium-driven Biolistic device, *Technique 3*:3-16)). Plasmids pZS223 and pZS224 are used as the coating plasmid DNA.

The bombarded tissue is then cultured for approximately 2 days on a cell division-promoting media, after which the plant tissue is transferred to a selective media containing an inhibitory amount of the particular selective agent. Transformed explants form green shoots in approximately 3-8 weeks. Leaves from these shoots are then subcultured on the same selective media to ensure production and selection of homoplasmic shoots.

### EXAMPLE 3. DNA GEL BLOT ANALYSIS OF TRANSPLASTOMIC LINES

Transformed plants selected for marker *aad*A marker gene expression are analyzed to determine whether the entire plastid content of the plant has been transformed (homoplastic transformants). Typically, following two rounds of shoot formation and spectinomycin selection, approximately 50% of the transgenic plantlets which are analyzed are homoplastic, as determined by Southern blot analysis of plastid DNA. Homoplasmic plantlets are selected for further cultivation.

Following a second round of shoot formation and spectinomycin selection, two transplastomic lines for each construct are obtained, Nt-pZS223 and Nt-pZS224. These lines are checked for homoplasmy. Southern blot analysis is used to confirm the integration of the chimeric cry genes in the tobacco plastid genome. Preparation, electrophoresis, and transfer of DNA to filters is as described (Svab *et al.,* (1993 supra)).

The complete disappearance of the 3.3 Kb native tobacco *Bam*HI fragment in the Nt-pZS223 and Nt-pZS224 transformants with a probe covering the region of integration, and the appearance of expected sized bands for the inserted DNA fragments in those transformants, 5.5 kb and 7.3 kb, respectively (see Fig. 1), establishes that the transformed plants are homoplasmic for the intended constructs. Probing identical filters with the *aad*A, *cry*IA(c) protoxin, and synthetic *cry*IA(c) genes demonstrated a linkage of the *aad*A and *cry*IA(c) genes to the expected 5.5 and 7.3 Kb *Bam*HI fragments as well as the lack of these genes in the negative control.

### EXAMPLE 4. INSECT BIOASSAYS

As described, the development of transformed plant lines Nt-pZS223 and Nt-pZS224 is accomplished on RMOP media supplemented with 500 mg/l Spectinomycin dihydrochloride. Plants are subcloned on the same selective medium, by the method according Svab *et al.* (1990 supra). Selected plants are then rooted in MS media containing 1 mg/l IBA, 500 mg/l Spectinomycin dihydrochloride and 0.6% phytagar.

*Helicoverpa zea* and *Heliothis virescens* eggs are obtained from the USDA-ARS in Stoneville, MS. and allowed to hatch. Neonate larva are placed on Tobacco Budworm Diet from Bioserve (Frenchtown, NJ), and incubated in a 16:8 photoperiod at 28°C for 5 days. The larva develop during this time to late second or early third instar.

At 5 days, fully expanded leaves are excised from the tobacco plants and placed on 3 ml of 2% agar in a 32 well rearing tray from CD International (Pitman, NJ). The larva are placed 1 per well, sealed and incubated for 5 days at the same conditions. At day 10, the leaf material consumed by the insect is estimated and insects checked for mortality. The larva are considered dead if they showed no movement after prodding with forceps.

### EXAMPLE 5. INSECTICIDAL FEEDING ACTIVITY

To determine the presence and relative amount of active *Bacillus thuringiensis* d-toxin in the tobacco lines homoplasmic for native protoxin and synthetic 'truncated' *cry*IA(c) gene expression constructs, efficacy of these plants to third instar *Heliothis virescens* (tobacco budworm) and *Helicoverpa zea* (corn earworm/cotton bollworm) larvae is tested. Both test insects are sensitive to the cryIA(c) toxin with *H. zea* being 10-fold more resistant than *H. virescens* (MacIntosh et *al.* (1990) *J. Invertebr. Pathol. 56*:258-266.).

Third instar larvae are chosen for the bioassay since the insects are more resistant to the toxin at this stage than are first instar larvae thus allowing a more stringent comparison between the control and test plants. Tobacco lines designated 4083 and 4084, derived by nuclear transformation with the same synthetic *cry*IA(c) gene as used in pZS223 and shown to be highly toxic to third instar *H*. *virescens* larvae, are used as positive controls in the bioassay. Nicotiana tabacum var. 'Petite Havana' serves as the negative control since this is the genetic background used to generate the transplastomic lines.

Table 1 is a summary of *Bacillus thuringiensis* tobacco insect feeding assays. The data demonstrates that transplastomic line Nt-pZS224 is very toxic to both *H*. *virescens* and *H. zea* as it causes 100% mortality to these insects while sustaining less than 2% total leaf damage. This result compares favorably to the results for positive control 4083 and 4084 tobacco plants. The 4083-2-4 plant when assayed with *H. zea* causes 100% mortality but sustains a much greater level of leaf feeding damage than the Nt-pZS224 tobacco line indicating less toxin production. Tobacco line 4084-4-1 performed comparably to Nt-pZS224 tobacco in feeding, although it does not compare to the levels of toxin produced in Nt-pZS224 when measured as a component of total leaf protein.

Tobacco line Nt-pZS223 shows no detectable bioactivity.

**TABLE 1**

| **SUMMARY OF BT TOBACCO INSECT FEEDING ASSAYS** | | | | | | |
|---|---|---|---|---|---|---|
| Chloroplast | Vector | plants tested | Heliothis .vtrescans" | % Leaf eaten | Heflocovsrpa zea^{∧∧} | % Leaf eatan |
| synthetic toxin gene | pZS223 | 223-3 | NO mortality | 100% | NO mortaliry | 100% |
| | | 223-5 | NO mortality | 75% | NO mortality | 100% |
| | | 223-12 | NT⁻ | | NO mortality | 100% |
| | | 223-13 | NO mortality | 75% | NT⁻ | |
| wild type protoxin gene | pZS224 | 224-5 | 100% mortality | 2% | 100% mortality | 2% |
| | | 224-9 | 100% mortality | 2% | 100% mortality | 2% |
| Nuclear Controls | | | | | | |
| synthetic toxin gene | pCGN4083 | 4083-1-2 | 100% mortality | 2% | NT⁺ | |
| | | 4083-2-4 | NT⁻ | | 100% mortality | 40% |
| | pCGN4084 | 4084-8-5 | 100% mortality | 2% | NT⁺ | |
| | | 4084-1-1 | NT⁻ | | 100% mortality | 2% |
| Untransformed Controls | | | | | | |
| | | control 1 | 25% mortality | 75% | NO mortality | 100% |
| | | control 2 | NO mortality | 100% | NT⁺ | |
| | | control 3 | 50% mortality | 75% | NT⁺ | |

| | | | | | | |
|---|---|---|---|---|---|---|
| ^{∧∧}10 third Instar larva were individually tested per plant *NT: Plant not tested | | | | | | |

All publications and patent applications mentioned in this specification are indicative of the level of skill of those skilled in the art to which this invention pertains.

## Claims

1. A construct comprising the following as operably joined components in the 5' to 3' direction of transcription:
(a) a promoter functional in a plant plastid;
(b) a native *Bacillus thuringiensis* DNA sequence encoding an insecticidal *Bacillus thuringiensis* toxin;
(c) a transcription termination region capable of terminating transcription in a plant plastid;
(d) a gene encoding a marker construct for selection of plant cells comprising a plastid expressing said marker; and
(e) DNA regions of homology to the genome of said plastid,
wherein said regions of homology in (e) flank said components (a), (b), (c), and (d) of said construct.

2. The construct according to claim 1 wherein said plant plastid is a chloroplast.

3. The construct according to claim 1 wherein said DNA encoding sequence encodes a protoxin.

4. The construct according to claim 1 wherein said DNA encoding sequence is the crylA(c) gene.

5. A plant cell plastid containing the construct according to claim 1.

6. A plant, plant seed, plant cell or progeny thereof containing the plant cell plastid according to claim 5.

7. The plant, plant seed, plant cell or progeny thereof according to claim 6 wherein said plant is tobacco.

8. A method for the expression of an insecticidal *Bacillus thuringiensis* toxin in a plant cell, wherein said method comprises expressing said *Bacillus thuringiensis* toxin in plastids of said plant cell and wherein said toxin is expressed from a construct as defined in any one of claims 1 to 4.

9. The method according to claim 8 wherein said plant plastids are chloroplasts.

## Patentansprüche

1. Konstrukt, das die folgenden Komponenten umfasst, die in der 5'→3'-Transcriptionsrichtung funktionell miteinander verknüpft sind:
(a) einen Promotor, der in einem Pflanzenplastid funktioniert;
(b) eine native *Bacillus-thuringiensis*-DNA-Sequenz, die ein insektizides *Bacillus-thuringiensis-Toxin* codiert;
(c) einen Transcriptionsterminationsbereich, der die Transcription in einem Pflanzenplastid beenden kann;
(d) ein Gen, das ein Markerkonstrukt für die Selektion von Pflanzenzellen codiert, die ein Plastid umfassen, das den Marker exprimiert; und
(e) DNA-Bereiche mit Homologie zum Genom des Plastids;
wobei die Bereiche mit Homologie in (e) die Komponenten (a), (b), (c) und (d) des Konstrukts flankieren.

2. Konstrukt gemäß Anspruch 1, wobei das Pflanzenplastid ein Chloroplast ist.

3. Konstrukt gemäß Anspruch 1, wobei die codierende DNA-Sequenz ein Protoxin codiert.

4. Konstrukt gemäß Anspruch 1, wobei es sich bei der codierenden DNA-Sequenz um das cryIA(c)-Gen handelt.

5. Pflanzenzellenplastid, das das Konstrukt gemäß Anspruch 1 enthält.

6. Pflanze, Pflanzensamen, Pflanzenzelle oder Nachkommen davon, die das Pflanzenzellenplastid gemäß Anspruch 5 enthalten.

7. Pflanze, Pflanzensamen, Pflanzenzelle oder Nachkommen davon gemäß Anspruch 6, wobei es sich bei der Pflanze um Tabak handelt.

8. Verfahren zur Expression eines insektiziden *Bacillus-thuringiensis*-Toxins in einer Pflanzenzelle, wobei das Verfahren das Exprimieren des *Bacillus-thuringiensis-Toxins* in Plastiden der Pflanzenzelle umfasst und wobei das Toxin aus einem Konstrukt exprimiert wird, wie es in einem der Ansprüche 1 bis 4 definiert ist.

9. Verfahren gemäß Anspruch 8, wobei die Pflanzenplastiden Chloroplasten sind.

## Revendications

1. Construction comprenant ce qui suit comme composants joints de façon opérationnelle dans la direction de transcription 5' à 3' :
(a) un promoteur fonctionnel dans un plaste végétal ;
(b) une séquence d'ADN natif de *Bacillus thuringiensis* codant une toxine de *Bacillus thuringiensis* insecticide ;
(c) une région de terminaison de transcription capable de terminer la transcription dans un plaste végétal ;
(d) un gène codant une construction marqueur pour la sélection de cellules végétales comprenant un plaste exprimant ledit marqueur ; et
(e) des régions d'ADN d'homologie au génome dudit plaste,
dans laquelle lesdites régions d'homologie en (e) flanquent lesdits composants (a), (b), (c), et (d) de ladite construction.

2. Construction selon la revendication 1, dans laquelle ledit plaste végétal est un chloroplaste.

3. Construction selon la revendication 1, dans laquelle ladite séquence codante d'ADN code une protoxine.

4. Construction selon la revendication 1, dans laquelle ladite séquence codante d'ADN est le gène *cry*IA(c).

5. Plaste de cellule végétale contenant la construction selon la revendication 1.

6. Végétal, graine de végétal, cellule végétale ou descendant de ceux-ci contenant le plaste de cellule végétale selon la revendication 5.

7. Végétal, graine de végétal, cellule végétale ou descendant de ceux-ci selon la revendication 6, dans lesquels ledit végétal est le tabac.

8. Procédé pour l'expression d'une toxine de *Bacillus thuringiensis* insecticide dans une cellule végétale, dans lequel ledit procédé comprend l'expression de ladite toxine de *Bacillus thuringiensis* dans les plastes de ladite cellule végétale et dans lequel ladite toxine est exprimée à partir d'une construction telle que définie dans l'une quelconque des revendications 1 à 4.

9. Procédé selon la revendication 8, dans lequel lesdits plastes végétaux sont des chloroplastes.
